# DEMANDE DE BREVET EUROPEEN

(11) **EP 4 079 342 A1**
(43) Date de publication de la demande: **26.10.2022**
(21) Numéro de dépôt: 22175964.0
(22) Date de dépôt: 19.12.2017
(51) Int. Cl.: A61L 27/36, C12M 3/00, C12M 1/00, B01L 1/04

(54) **PROCÉDÉ AUTOMATISÉ DE PRODUCTION DE TISSUS HUMAINS OU ANIMAUX POUR GREFFES**

(30) Priorité: 26.12.2016 BE 201605978
(62) Demande divisionnaire de: 17825201.1
(71) Demandeur: Theracell Consulting SPRL, 1380 Lasne (BE)
(72) Inventeur: DUFRANE, Denis, 1380 Lasne (BE)
(74) Mandataire: Willnegger, Eva

(57) **Abrégé**

La présente invention propose un procédé d'obtention d'une matrice tissulaire pour allogreffe ou xénogreffe, selon lequel, après récupération d'un tissu biologique on classe ledit tissu, on traite ledit tissu et on conditionne la matrice tissulaire obtenue, toutes ces étapes étant mises en œuvre de façon automatisée à l'intérieur d'un même réacteur « classé ». L'invention propose également le réacteur permettant de mettre en œuvre le procédé de l'invention.

## Description

La présente demande concerne le domaine de la production de tissus humains ou animaux, en vue de leur utilisation pour des greffes (allogreffes ou xénogreffes).

Une allogreffe implique un donneur et un receveur humain. Une autogreffe implique un seul être humain.

Une xénogreffe implique un donneur animal et un receveur humain.

Un nombre grandissant de troubles musculo-squelettiques humains nécessitent des traitements par reconstruction ou substitution osseuse, aussi bien dans le domaine dentaire (reconstruction maxillo-faciale par implant dentaire), qu'en cancérologie (suite à l'ablation de tumeurs osseuses) ou en chirurgie orthopédique (suite à une fracture ou pour la fusion de vertèbres).

Actuellement, la reconstruction osseuse se fait par autogreffe, généralement par ponction d'os au niveau de la crête iliaque de la personne qui nécessite le traitement. Cependant, en plus d'être douloureuse, cette ponction n'est pas réversible. En effet, l'os iliaque ne se régénère pas, et la quantité de tissu osseux disponible est donc limitée. L'autogreffe nécessite, en outre, une double intervention chirurgicale sur un même individu, ce qui a également un impact non négligeable sur la récupération postopératoire.

Une autre approche est l'utilisation de substituts osseux, largement accessibles et peu coûteux, comme par exemple des prothèses en céramique, corail ou verre. Malheureusement, du fait de la faible réglementation sur ce marché, de nombreux problèmes de fiabilité, de compatibilité de matériaux, voire même de toxicité sont à l'origine de nombreux problèmes post-opératoires et le personnel médical est de plus en plus méfiant vis-à-vis de ces substituts.

L'allogreffe est également pratiquée. En pratique, du tissu osseux est enlevé à un individu, par exemple une tête fémorale ôtée lors de la pose d'une prothèse de hanche. Ce tissu contient de la matrice osseuse ainsi que des cellules du donneur. Afin d'éliminer le risque de rejet, ou réponse inflammatoire, lors de la transplantation, il est nécessaire de traiter le tissu afin de le nettoyer de toutes traces « génétiques » du donneur, c'est-à-dire d'en éliminer les traces de cellules, de sang ou de graisse.

La méthode de nettoyage ou de « décellularisation » du tissu osseux pour donner une matrice osseuse decellularisée est décrite dans Dufrane et al., ; Biomaterials. 2002 Jul;23(14):2979-88 et Dufrane et al. Eur Cell Mater. 2001 Jan 10;1:52-8; discussion 58.

Les principales étapes de cette méthode sont :
- Centrifugation du tissu prélevé du donneur afin d'en éliminer le sang et la graisse ;
- Découpe du tissu, généralement en cubes de taille variable ;
- Traitement chimique, afin d'en éliminer les traces de cellules, et d'inactiver les virus et/ou bactéries ;
- Lyophilisation, afin d'obtenir de la matrice osseuse déminéralisée stable,
- Emballage.

Bien que l'allogreffe soit à ce jour la technique de reconstruction osseuse la plus fiable, l'accès des patients à cette technique est fortement restreint par la méthode et les coûts de production de la matrice osseuse décellularisée.

En effet, actuellement, toutes ces étapes sont réalisées manuellement, par du personnel hospitalier, en salle blanche stérile dite « classée », c'est-à-dire construite suivant un certain nombre de normes et faisant l'objet de contrôles stricts. Ces étapes doivent tout d'abord être réalisées chacune dans des enceintes stériles classées différentes, ou isolateurs, au sein de la zone de production, chaque isolateur contenant les équipements adéquats pour chaque étape, et devant être maintenus stériles. Il en résulte un grand espace mort pour le déplacement du personnel entre chaque isolateur. Il est également nécessaire de prendre des précautions particulières pour le transfert des lots de production d'un isolateur à l'autre.

Il faut ensuite assurer la traçabilité des lots quant à l'origine du tissu. Les erreurs de manipulation par le personnel ne sont pas à exclure et peuvent conduire à l'exclusion de lots.

L'aspect financier est en outre également non négligeable. En effet, la production d'un lot prend plusieurs semaines, cette durée pouvant être influencée par la disponibilité du personnel hospitalier qui doit assurer ses activités principales de soins. La demande en temps de travail, ainsi qu'en espace dédié hautement réglementé, représente pour un établissement hospitalier un coût important. Pour ces raisons, de nombreux établissements hospitaliers n'investissent pas dans la production de matrices osseuses décellularisées. L'accès à cette source osseuse pour l'allogreffe est donc actuellement très limité.

Pour résumer, les problèmes rencontrés sont liés au fait que :
- la production se fait manuellement, ce qui implique :
   o une classification relative des différentes étapes,
   o un besoin d'espace de travail conséquent,
   o de la manutention et
   o des difficultés de traçabilité ;
- la manipulation par des opérateurs peut être source d'erreurs et affecter la qualité du produit et
- la durée de la production engendre des coûts prohibitifs.

Par classification relative, il faut comprendre ici le maintien du tissu biologique dans des conditions stériles, non seulement durant chaque étape, mais également entre chaque étape, lors d'un éventuel déplacement physique du tissu entre deux zones stériles.

Ces problèmes de production ne sont pas limités aux tissus osseux, mais sont également rencontrés pour d'autres tissus biologiques utilisés en allogreffe, comme la peau, les tendons, la vessie,... ou le tissu adipeux. Ces mêmes problèmes sont également rencontrés pour la production des thérapies cellulaires.

Afin d'améliorer l'accès des patients à cette source de tissu biologique fiable pour des allogreffes, la demanderesse a mis au point un procédé résolvant les problèmes cités ci-dessus.

### Solution de l'invention

La présente invention propose, à cet effet, un procédé d'obtention d'une matrice tissulaire pour allogreffe ou xénogreffe, selon lequel, après récupération d'un tissu biologique :
- on classe ledit tissu,
- on traite ledit tissu et
- on conditionne la matrice tissulaire obtenue,
caractérisé par le fait que ces étapes sont mises en œuvre de façon automatisée à l'intérieur d'un même réacteur « classé ».

Avec audace, la demanderesse a sélectionné, pour résoudre l'ensemble des problèmes, l'un de ceux-ci, et en l'occurrence, celui dont il était a priori le plus difficile de se débarrasser, mais a réalisé qu'en recherchant à éliminer un maximum la classification, elle s'affranchissait de toutes les autres difficultés. A cet effet, la présente demande concerne une invention de problème.

Par « classer » le tissu, il faut comprendre le préparer pour l'introduire en environnement stérile répondant aux normes requises pour le procédé. De même, le réacteur « classé » est un réacteur répondant à ces mêmes normes.

Le conditionnement fait référence à toute forme d'emballage, comme par exemple l'introduction de la matrice tissulaire dans une poche plastique scellée sous vide, ou l'introduction de la matrice tissulaire dans une seringue prête à l'emploi, elle-même emballée de façon stérile sous plastique.

Pour certains tissus biologiques, le procédé de l'invention peut comprendre une étape de découpe ou de broyage du tissu. Il peut, en outre, également comprendre une étape de séchage ou de lyophilisation préalable au conditionnement.

Le traitement du tissu peut comprendre une ou plusieurs des étapes suivantes :
- centrifugation du tissu ;
- traitement chimique ;
- traitement biologique.

Un traitement chimique comprend toute exposition du tissu biologique à des agents ou réactifs chimiques, par exemple l'agitation du tissu dans une solution contenant un agent antibactérien ou une solution dont le pH est ajusté acide ou basique, par ajout de composés ioniques. Le tissu biologique peut subir plusieurs traitements chimiques consécutifs ayant des finalités différentes, comme par exemple le dégraissage ou l'inactivation des bactéries ou des virus ou du prion ou encore la déminéralisation pour augmenter l'ostéoconduction.

Un traitement biologique fait référence à l'application d'agents biologiques au tissu concerné, comme par exemple des facteurs de croissance ou des protéines induisant une différenciation cellulaire.

L'invention concerne également le réacteur classé pour la mise en œuvre du procédé de l'invention, réacteur comprenant
- un sas d'introduction et de classement d'un tissu biologique ;
- des moyens de traitement du tissu biologique agencés pour produire une matrice tissulaire ;
- des moyens de conditionnement de la matrice tissulaire produite ;
- un sas de sortie de la matrice tissulaire conditionnée ;
- des moyens robotiques de déplacement du tissu biologique du sas d'introduction et de classement aux moyens de traitement du tissu biologique, et
- des moyens robotiques de déplacement de la matrice tissulaire des moyens de traitement aux moyens de conditionnement, puis des moyens de conditionnement au sas de sortie.

Le sas d'introduction et de classement d'un tissu biologique, de même que le sas de sortie de la matrice tissulaire conditionnée sont agencés pour maintenir à l'intérieur du réacteur les conditions stériles répondant aux normes requises pour le procédé. Le classement du tissu ne se fait donc qu'au niveau du sas d'introduction. Le sas de sortie est bien évidemment également prévu que les conditions stériles soient maintenues lors de la sortie du produit. Le réacteur classé peut ainsi être agencée de façon compacte, ne permettant pas forcément qu'un individu puisse y pénétrer. Toutes les étapes du procédé de l'invention peuvent ainsi y être mises en œuvre selon une séquence optimisée.

L'invention sera mieux comprise à l'aide de la description suivante de plusieurs mises en oeuvre de l'invention, en référence au dessin en annexe, sur lequel :
- la figure 1 est une illustration schématique du procédé de l'invention,
- la figure 2 est une illustration schématique d'un réacteur pour la mise en œuvre de l'invention, et
- la figure 3 représente, en perspective, l'intérieur d'un réacteur selon l'invention.

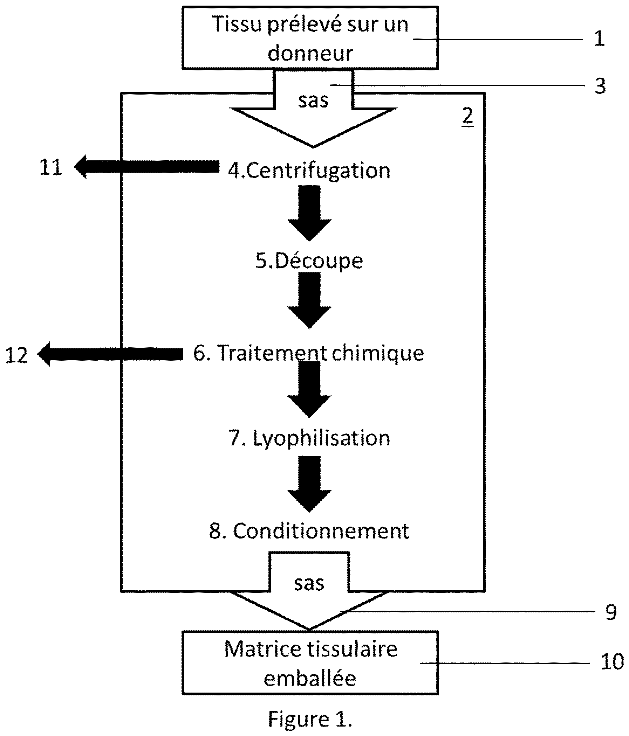

En référence à la figure 1, un tissu biologique 1, prélevé sur un donneur, est introduit dans un réacteur 2 via un sas d'entrée 3. Le tissu subit un certain nombre d'étapes : centrifugation 4, découpe 5, traitement chimique 6, lyophilisation 7 et conditionnement 8. La matrice tissulaire emballée 10 qui en résulte sort ensuite du réacteur 2 via un sas de sortie 9.

Le tissu 1 peut par exemple être une tête fémorale, prélevée sur un individu donneur, lors de la pose d'une prothèse de hanche. Cette tête fémorale brute, pour pouvoir servir à la fabrication de matrice osseuse en vue de son utilisation pour une reconstruction osseuse chez un autre patient, doit subir un certain nombre de transformations, dans un environnement stérile contrôlé. La tête fémorale va donc être, en premier lieu, introduite dans le réacteur 2 dit « classé », dans le sens où il y règne un environnement stérile correspondant aux normes requises pour ces transformations.

L'introduction de la tête fémorale 1 se fait via le sas d'entrée 3, dont la configuration est telle qu'aucune contamination provenant de l'extérieur ne puisse pénétrer dans le réacteur 2. Ce sas 3 peut par exemple être muni d'un système de double porte, chaque porte s'ouvrant alternativement, et d'un flux laminaire d'air classique, orienté vers l'extérieur du sas. Lors de son introduction, il est envisageable de placer la tête fémorale dans un récipient particulier qui va ensuite faciliter sa prise en charge au cours des étapes du procédé. Le sas 3 permet ainsi d'introduire et de classer le tissu biologique, ici la tête fémorale 1, entrant dans le réacteur 2.

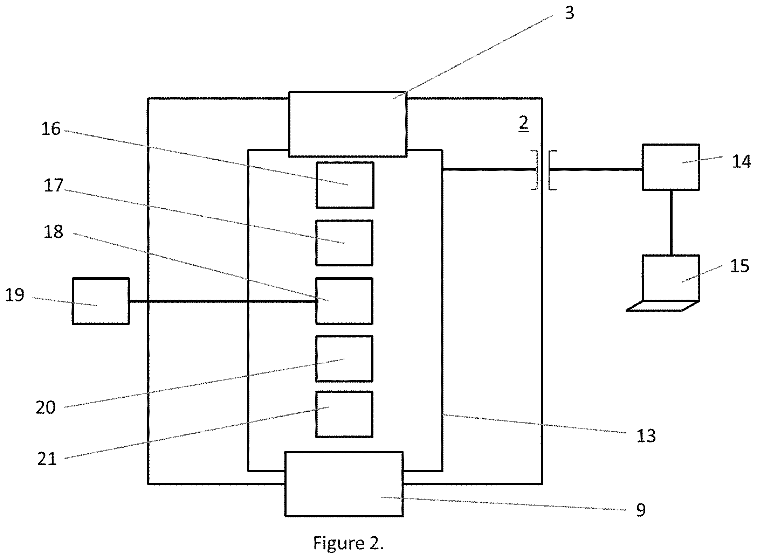

En référence à la figure 2, à l'intérieur du réacteur 2, un automate 13, ou une série d'automates coopérant entre eux, comprenant des unités de fonctions diverses, est agencé pour permettre la mise en œuvre des étapes du procédé. Cet automate 13 est avantageusement piloté par un système informatique 14 auquel des instructions auront éventuellement été données par un opérateur, au moyen d'un poste de pilotage 15, ici un ordinateur. Le réacteur peut prendre diverses formes et avoir des tailles variées. Il peut être par exemple construit en métal, comme un container de transport routier ou maritime, ou avoir une partie de ses parois vitrées. Les seules limitations sur l'aspect de ce réacteur sont liées à la contrainte de pouvoir y établir et y maintenir un environnement stérile répondant aux normes requises pour le procédé de fabrication. Ces contraintes et ces normes peuvent varier selon les zones géographiques. Un certain nombre d'éléments peuvent être intégrés à ce réacteur afin d'assurer l'environnement stérile, comme par exemple des unités de ventilation, de décontamination ou de climatisation.

Ici, l'automate 13 introduit la tête fémorale 1, éventuellement avec son récipient, dans une unité de centrifugation 16 où elle est centrifugée, afin d'en séparer la matrice osseuse des résidus indésirables 11, tels que la moelle, le sang ou des résidus graisseux. La tête fémorale 1, ainsi nettoyée, est ensuite transférée vers une unité de découpe 17, configurée pour produire des morceaux d'os à un calibre prédéfini. Ces morceaux sont ensuite introduits dans une enceinte contenant un bain de traitement chimique 18, où ils vont être agités pendant un certain temps prédéfini. Le bain de traitement peut par exemple être une solution désinfectante pour inactiver les bactéries ou tout autre bain adapté à la nature du tissu traité.

L'enceinte 18 dans laquelle est réalisé le traitement chimique peut être agencée de façon à ce qu'à la fin du traitement, la solution chimique usagée 12 puisse être évacuée vers un conteneur 19 adapté annexé au réacteur 2. Il est envisageable, par exemple, que ce conteneur soit situé à l'extérieur et isolé du réacteur à l'aide de valves, afin de pouvoir le remplacer lorsqu'il est plein, sans affecter l'environnement stérile régnant dans le réacteur. Il est envisageable d'éliminer les résidus issus de la centrifugation, de la même façon. Il est également envisageable de connecter des conteneurs de solutions « propres » qui serviront à alimenter les bains chimiques. Le contenu de ces conteneurs est facilement traçable par le système informatique 14, par exemple via un système de code-barres.

Plusieurs traitements chimiques successifs peuvent avoir lieu sur les morceaux d'os, au sein de la même enceinte de traitement, ou dans des enceintes séparées à l'intérieur du réacteur 2. Ils peuvent être complétés par des étapes de rinçage. Les morceaux de matrice osseuse décellularisée ainsi traités sont ensuite lyophilisés dans un lyophilisateur 20, c'est-à-dire séchés jusqu'à ce qu'ils contiennent, par exemple, moins de 5% d'humidité, puis emballés sous vide ou sous atmosphère protectrice dans une unité d'emballage 21. Cela assure à la matrice osseuse 10 une stabilité optimale permettant leur conservation à température ambiante pendant plusieurs mois. Il est envisageable d'inclure une étape d'étiquetage des emballages afin d'assurer la traçabilité des lots produits. La matrice tissulaire emballée 10 est extraite du réacteur 2 par le sas de sortie 9, similaire au sas d'entrée 3. Il est même possible de configurer le réacteur 2 pour qu'il n'ait qu'un seul sas d'entrée et de sortie.

Le poste de commande, de pilotage 15 comprend le système informatique 14 sur lequel est renseigné le procédé de fabrication du tissu emballé 10. Le système informatique 14 pilote l'automate 13 dans le réacteur 2. Certains paramètres peuvent devoir être renseignés par l'opérateur, comme par exemple la nature du tissu, si l'automate peut gérer la production de plusieurs types de tissus, les durées des traitements chimiques ou le pourcentage d'humidité désiré dans la matrice tissulaire emballée 10.

Le procédé de fabrication décrit ci-dessus permet donc d'introduire un tissu biologique « brut » dans un réacteur et de récupérer en sortie un tissu biologique décontaminé et prêt à être utilisé pour une greffe. Il n'y a dans ce procédé qu'une seule étape de classification, à l'entrée du tissu 1 dans le réacteur 2. La continuité de la classification est assurée par l'espace restreint et contrôlé du réacteur dont le tissu ne sort pas avant la fin de la production. Aucun opérateur n'ayant à intervenir durant le déroulement du procédé, les erreurs de manipulation sont exclues, la traçabilité est assurée, les besoins en espace et en manutention sont réduits au strict minimum. Il en résulte que les coûts de production sont également optimisés.

Un autre avantage du procédé de l'invention est la possibilité de gérer des « petites » productions, en continu. Usuellement, lorsque la production est réalisée manuellement dans un centre hospitalier (via une Banque de tissus), le personnel hospitalier attend d'avoir une quantité suffisante de matière de départ, plusieurs dizaines de têtes fémorales, par exemple 48, avant de commencer une production et, ce, afin d'optimiser les coûts. Le procédé de l'invention permet de commencer une production avec une seule tête fémorale et d'assurer la production en continu, ou à chaque fois qu'une nouvelle tête fémorale est disponible.

Le réacteur 2 peut-être avantageusement agencé pour permettre la production simultanée, en parallèle ou en décalé, de plusieurs lots de matrice tissulaire. C'est-à-dire que deux tissus biologiques bruts peuvent être introduits et traités en même temps, ou qu'un second tissu biologique peut être introduit alors qu'un premier tissu est déjà en cours de traitement. La traçabilité des lots de matrice tissulaire est assurée pour chaque tissu biologique.

Il est évident pour l'homme du métier que le tissu 1 peut également être prélevé sur un donneur animal. Le traitement de têtes fémorales a été ici décrit, mais il pourrait également s'agir du traitement de tout autre tissu osseux, de peau ou de tout autre tissu envisageable pour l'homme du métier.

Le procédé de l'invention ne se limite pas à la production de matrices tissulaires. Les thérapies cellulaires, ou biothérapies, peuvent également profiter du procédé de l'invention. Ces thérapies sont une forme d'autogreffe fréquemment utilisée en cancérologie et où des cellules différenciées particulières sont produites à partir de cellules souches prélevées d'un individu pour lui être réimplantées dans l'organe à traiter. La préparation de ces thérapies, jusqu'à présent réalisée manuellement, souffre des mêmes problèmes que la production de matrices tissulaires. Leur fabrication comprend également des étapes de classement, de traitement et de conditionnement.

Idéalement, le réacteur 2 est facilement transportable par tout moyen de transport, et peut être installé facilement partout dans le monde. Le procédé de l'invention ne nécessite pas l'intervention de personnel hospitalier hautement qualifié et peut être adapté aux normes en vigueur là où il est mis en œuvre.

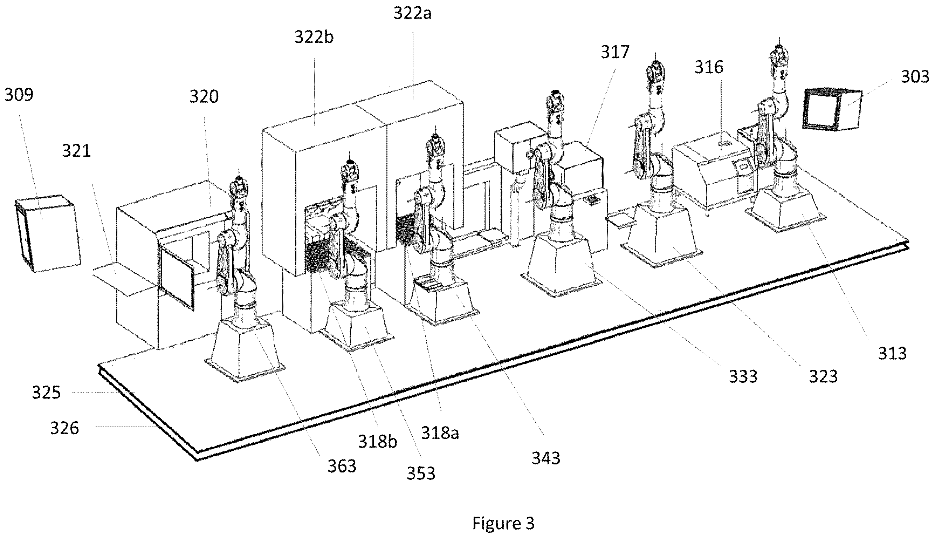

Une forme de réalisation particulière du réacteur de l'invention est détaillée sur la figure 3, représentant l'agencement intérieur d'un réacteur de l'invention. Les parois délimitant le réacteur ne sont pas représentées, pour des raisons de clarté, mais il est bien évident qu'elles entourent, à la façon d'un conteneur scellé, les équipements contenus dans le réacteur. Par scellé, il est entendu que le réacteur ne comprend aucune ouverture, autre que les sas et ouvertures spécifiquement prévues pour des besoins techniques.

Le réacteur 302 comprend un sas 303 d'introduction et de classement de tissus biologiques, prélevés sur un donneur et un sas 309 de sortie de matrice tissulaire conditionnée, chaque sas étant ici placé à une extrémité du réacteur. Des pots 327, contenant des tissus biologiques, sont ici représentés dans le sas 303. Entre les deux sas sont placés, côte à côte, cinq équipements: une centrifugeuse 316, une unité 317 de découpe, deux stations 318a et 318b de traitement chimique, placées chacune sous une hotte 322a et 322b, un lyophilisateur 320 et une station d'emballage 321, devant lesquels sont positionnés respectivement cinq bras robotiques articulés 313, 323, 343, 353 et 363 ainsi qu'un bras robotique articulé 333 positionné entre l'unité de découpe 317 et la station de traitement chimique 318a. Tous les éléments décrits ci-dessus sont placés sur un plancher 325 sous lequel est prévu un espace 326.

Les éléments du réacteur 302 ayant été décrits, l'articulation des éléments entre eux pour la mise en œuvre du procédé de l'invention vont maintenant être détaillés.

Le sas 303 d'introduction et de classement de tissus biologiques est similaire au sas 3 des figures 1 et 2. Après avoir ouvert la porte externe du sas, un opérateur dépose dans le sas 303 un tissu biologique à traiter, dans un pot 327. Le flux d'air à l'intérieur du sas est tel qu'aucune contamination extérieure ne peut pénétrer dans le sas, jusqu'à ce que la porte externe soit refermée.

Le procédé automatisé de traitement débute par l'ouverture de la porte intérieure (au réacteur) du sas 303. Le bras robotique 313 vient saisir le pot 327 dans le sas 303 et le dépose dans une cavité de la centrifugeuse 316. Le pot 327 peut par exemple être agencé pour correspondre aux cavités de travail de la centrifugeuse. Il peut avoir une double paroi, la paroi intérieure étant ajourée, afin que les substances extraites du tissu biologique durant la centrifugation traversent la paroi ajourée et puissent ainsi être physiquement séparée du tissu biologique. Plusieurs lots, dans plusieurs pots peuvent éventuellement être centrifugés simultanément. Il est aisé de suivre, au cours du procédé, la position de chaque lot, dans tous les équipements, sans erreur possible.

Après centrifugation, le bras robotique 313 sort le pot 327 de la centrifugeuse 316. Le bras 323 vient y prélever le tissu biologique débarrassé, au moins en partie, d'éléments indésirables, pour procéder à sa découpe.

Il est également possible de prévoir une plateforme intermédiaire, ou les pots contenant le tissu biologique centrifugés sont entreposés avant d'être pris en charge par le bras 323.

La découpe peut se faire de façon traditionnelle, à l'aide d'une scie à ruban ou d'une fraise. Ces méthodes présentent néanmoins plusieurs inconvénients. Tout d'abord, le frottement avec le tissu biologique génère de la chaleur, généralement au-dessus de 47 °C, qui abime ce tissu. En particulier lorsqu'il s'agit d'un os, les cavités de l'os s'obstruent à cause de la chaleur et de la pression exercée à la surface de découpe. Ensuite, il faut, en théorie, changer le ruban de coupe pour éviter toute contamination entre lots, ce qui est rarement fait lorsque le procédé est effectué par des techniciens, dans un hôpital. Enfin, ces méthodes, peu flexibles géométriquement (machines 4 axes), génèrent beaucoup de perte du tissu biologique.

Pour pallier ces inconvénients, la demanderesse a, astucieusement, intégré dans le procédé automatisé, une découpe par jet à haute pression d'eau, non abrasif. Cette technique a déjà été utilisée, de façon manuelle par des chirurgiens en salle d'opération, comme cela est décrit dans le Journal of The Mechanical Behavior of Biomedical Materials, 62, 2016, 495-503, pour couper partiellement des os au cours d'une opération. L'utilisation d'un jet d'eau à haute pression permet d'une part de couper très précisément et très nettement, sans élévation de température pouvant endommager les tissus, et, d'autre part, de ne pas contaminer les lots entre eux. Un autre avantage lié à l'utilisation d'un jet d'eau à haute pression est de pouvoir fonctionner pour n'importe quel tissu, que ce soit de l'os ou de la peau. La pression du jet d'eau peut être adaptée au tissu à découper. La découpe est nette, avec une précision micrométrique. Lorsqu'il s'agit d'os, les alvéoles osseuses à l'interface de découpe ne sont pas endommagées.

En pratique, le bras 323 maintenant le tissu biologique à découper est mobile selon plusieurs axes simultanément et vient positionner et déplacer le tissu à découper sous le jet, selon n'importe quelle orientation prédéfinie. Le bras articulé étant mobile selon plusieurs axes, il est ainsi possible d'obtenir n'importe quelle géométrie de découpe, ce qui permet également d'optimiser la découpe afin de limiter les déchets et d'obtenir un rendement plus élevé de matrice tissulaire en fin de procédé.

Le jet d'eau sous pression est généré dans un équipement bien connu de l'homme du métier, comprenant un compresseur et une buse de sortie, par exemple en saphir ou diamant pour en limiter l'usure et de diamètre ajustable pour ajuster la pression et la précision de découpe.

Bien que la découpe au jet d'eau à haute pression soit techniquement plus performante, l'invention ne se limite pas à ce mode de découpe et tous les moyens classiques de découpent peuvent ici être utilisés.

Une fois découpé, les morceaux de tissu sont récupérés, par exemple sur une grille, avant d'être prélevés, un par un, par le bras 333, qui les dispose dans un panier, lot par lot, afin de ne pas mélanger des morceaux provenant de lots différents et d'assurer une traçabilité complète.

Le bras 343 introduit les paniers dans les bains de traitement chimique, comme décrits plus haut, puis les en ressort une fois le traitement fini. Il peut également gérer des étapes intermédiaires ou plusieurs traitements chimiques successifs différents, sur les plateformes 318a et 318b. Les hottes 322a et 322b sont des hottes classiques de laboratoire, permettant de réguler la qualité de l'air dans le réacteur au-dessus des solutions chimiques. Une fois le traitement chimique terminé, le bras robotique retire les paniers et les dépose à proximité du bras robotique 353.

Le bras robotique 353 prend les morceaux de tissus biologique traités un par un pour les disposer chacun dans un tube pré-scellé, c'est-à-dire un tube dont les capuchons sont déjà disposés sans toutefois être scellés, puis introduit chaque tube dans le lyophilisateur 320. A la fin de la lyophilisation, avant réouverture du lyophilisateur, une plaque vient appuyer sur les capuchons afin de sceller les tubes comprenant chacun un morceau de matrice tissulaire, prêt à être utilisé pour une greffe.

Le bras 363 va ensuite terminer le conditionnement des tubes, en y déposant par exemple une étiquette comprenant toutes les informations nécessaires à la traçabilité du lot, puis déposer les tubes de matrice tissulaire conditionnée dans le sas 309 de sortie afin qu'ils soient récupérés par un opérateur.

L'espace 326 prévu sous le plancher 325 peut permettre de faire passer tous les câbles et tuyaux nécessaires au raccordement, électriques, informatiques et fluidiques, des équipements ainsi qu'à l'évacuation des déchets générés au cours du procédé.

Les bras robotiques peuvent bien sur effectuer d'autres mouvements que ceux décrits ci-dessus, pour assurer le bon fonctionnement de l'ensemble du procédé. La disposition des équipements est ici décrite en ligne, mais toute autre disposition permettant la mise en œuvre du procédé de l'invention est envisageable. Il est notamment possible d'utiliser moins de bras robotiques voire un seul bras robotique effectuant toutes les opérations, entre des équipements qui seraient répartis autour de lui.

Les moyens robotiques, décrits plus hauts, sont ici illustrés par un ou plusieurs bras robotiques, fixés au sol. Néanmoins, tout autre configuration ou équipement équivalent peut être envisagé, que ce soit des moyens suspendus, et/ou sur rail, ayant des capacités de préhension ou étant spécifiquement configurés pour un matériel précis. La notion de bras robotique est à prendre au sens très large d'un moyen automatisé, programmable et/ou pilotable à distance. Une bande transporteuse peut éventuellement également être utilisée pour assurer le déplacement des tissus, matrice, déchets ou tout autre élément devant être déplacé.

Les équipements ici décrits sont des équipements classiques tels qu'une centrifugeuse ou un lyophilisateur. Selon le tissu traité, d'autres types d'équipements peuvent être utilisés ou des équipements su mesure peuvent être développés. Les moyens de traitement, de découpe et de conditionnement sont à prendre au sens large et comprennent tous les moyens connus de l'homme du métier.

## Revendications

1. Procédé d'obtention d'une matrice tissulaire (10) pour allogreffe ou xénogreffe, selon lequel, après récupération d'un tissu biologique (1) :
- on classe ledit tissu ;
- on traite ledit tissu, et
- on conditionne la matrice tissulaire obtenue ;
**caractérisé par le fait que** ces étapes sont mises en œuvre de façon automatisée à l'intérieur d'un même réacteur « classé » (2).

2. Procédé selon la revendication 1, comprenant une étape de découpe du tissu (5).

3. Procédé selon l'une des revendications 1 et 2, comprenant une étape de lyophilisation (7) préalable au conditionnement (8).

4. Procédé selon l'une des revendications 1 à 3, dans lequel le traitement du tissu (1) comprend au moins l'une des étapes du groupe comportant les étapes de :
- centrifugation du tissu (4),
- traitement chimique (6),
- traitement biologique.

5. Procédé selon l'une des revendications 1 à 4, dans lequel la matrice tissulaire (10) est une matrice osseuse décellularisée.

6. Procédé selon l'une des revendications 1 à 4, dans lequel la matrice tissulaire (10) est une thérapie cellulaire.

7. Procédé selon la revendication 2, dans lequel la découpe du tissu se fait par jet d'eau à haute pression.

8. Réacteur pour la mise en œuvre du procédé des revendications 1 à 7, comprenant
- un sas d'introduction et de classement d'un tissu biologique ;
- des moyens de traitement du tissu biologique agencés pour produire une matrice tissulaire ;
- des moyens de conditionnement de la matrice tissulaire produite ;
- un sas de sortie de la matrice tissulaire conditionnée et
- des moyens robotiques de déplacement du tissu biologique du sas d'introduction aux moyens de traitement du tissu biologique et des moyens robotiques de déplacement de la matrice tissulaire des moyens de traitement aux moyens de conditionnement puis au sas de sortie.

9. Réacteur selon la revendication 8, comprenant également des moyens de découpe du tissu biologique.

10. Réacteur selon la revendication 9, dans lequel les moyens de découpe comprennent un jet d'eau à haute pression, non abrasif.

11. Réacteur selon l'une des revendications 8 à 10, dans lequel les moyens robotiques de déplacement du tissu biologique et de la matrice tissulaire comprennent des bras robotiques.
